# FASCICULE DE BREVET EUROPEEN

(11) **EP 2 953 937 B1**
(45) Date de publication et mention de la délivrance du brevet: **27.09.2017**
(21) Numéro de dépôt: 14703848.3
(22) Date de dépôt: 11.02.2014
(51) Int. Cl.: C07D 307/68, B01J 23/644, B01J 21/18

(54) **PROCÉDÉ DE PRÉPARATION D'ACIDE 2,5-FURANE DICARBOXYLIQUE**
VERFAHREN ZUR HERSTELLUNG VON 2,5-FURANDICARBONSÄURE
METHOD FOR PREPARING 2,5-FURANDICARBOXYLIC ACID

(30) Priorité: 11.02.2013 FR 1351138
(43) Date de publication de la demande: 16.12.2015
(73) Titulaire: Centre National de la Recherche Scientifique (C.N.R.S.), 75016 Paris (FR); Université Claude Bernard Lyon 1, 69100 Villeurbanne (FR)
(72) Inventeur: BESSON, Michèle, F-01700 Les Echets (FR); ESSAYEM, Nadine, F-38540 Saint Just Chaleyssin (FR); AIT RASS, Hicham, F-60000 Beauvais (FR)
(74) Mandataire: Lavoix
(86) Numéro de dépôt international: PCT/EP2014/052622
(87) Numéro de publication internationale: WO 2014/122319

(56) Documents cités:
- WO-A1-2012/017052
- FR-A1- 2 669 634
- US-A- 2 407 066
- US-A1- 2008 103 318
- Peter Vinke: "Oxidation of carbohydrates and derivatives using carbon supported noble metal catalysts", , 21 novembre 1991 (1991-11-21), pages 1-151, XP055087077, Delft, NL Extrait de l'Internet: URL:http://repository.tudelft.nl/view/ir/u uid:c4187a70-7b13-4908-bca9-ea5f8da51409/ [extrait le 2013-11-07]
- KROEGER M ET AL: "A new approach for the production of 2,5-furandicarboxylic acid by in situ oxidation of 5-hydroxymethylfurfural starting from fructose", TOPICS IN CATALYSIS, BALTZER SCIENCE PUBLISHERS, BUSSUM, NL, vol. 13, 1 janvier 2000 (2000-01-01), pages 237-242, XP002471377, ISSN: 1022-5528, DOI: 10.1023/A:1009017929727 cité dans la demande
- MICHAEL A LILGA ET AL: "Production of Oxidized Derivatives of 5-Hydroxymethylfurfural (HMF)", TOPICS IN CATALYSIS, KLUWER ACADEMIC PUBLISHERS-PLENUM PUBLISHERS, NE, vol. 53, no. 15-18, 28 mai 2010 (2010-05-28), pages 1264-1269, XP019831620, ISSN: 1572-9028

## Description

La présente invention concerne un procédé de préparation d'acide 2,5-furane dicarboxylique (FDCA) par oxydation du 5-hydroxyméthylfurfural (HMF).

Le FDCA est un composé important pour l'industrie chimique, notamment pour l'industrie des polymères. En effet, le FDCA peut constituer un monomère de base notamment pour la préparation de polyamide ou polyester.

Le FDCA est notamment un monomère de base pour la préparation de polyéthylène furandicarboxylate (PEF). Or le PEF est décrit comme pouvant être un polymère bio-sourcé pouvant remplacer le polyéthylène téréphtalate (PET) (Energy Environ. Sci., 2012, 5, 6407-6422) obtenu à partir d'acide téréphtalique issu de ressources fossiles.

Ainsi, le FDCA obtenu à partir de l'HMF qui peut lui-même être obtenu par déshydratation de fructose est un composé bio-sourcé de choix qui pourrait remplacer des composés issus de ressources fossiles.

Il existe donc un intérêt important à fournir un procédé de préparation du FDCA à partir d'HMF.

Il est connu différents procédés (US2008/0103318 et WO2012/017052) de préparation de FDCA par oxydation du HMF en solution aqueuse alcaline en présence d'un catalyseur platine supporté, notamment platine supporté sur charbon, et d'une base faible. Dans le premier procédé, des rapports molaires HMF/Pt faibles sont nécessaires (utilisation de quantités importantes de platine) en réacteur continu pour un bon rendement, ce qui augmente le coût de mise en oeuvre du procédé. Dans le deuxième procédé, l'oxydant est l'oxygène pur sous pression et le réacteur est semi-fermé.

Il a été proposé de modifier le catalyseur en ajoutant du plomb (FR2669634 et Verdeguer et al., Journal of Molecular Catalysis, 1993, 85, 327-344). Cependant, les procédés sont alors menés en présence de base forte. De plus, les quantités de plomb ajoutées sont élevées et ne sont pas complètement déposées sur le solide catalytique, ce qui peut engendrer un problème de pollution non négligeable.

Etant donné l'instabilité du HMF dans le milieu aqueux acide de sa synthèse, il a également été proposé un procédé de préparation de FDCA à partir de fructose dans lequel l'étape d'oxydation du HMF, obtenu préalablement par déshydratation du fructose en présence de catalyseur acide, en FDCA, est réalisée dans un milieu solvant organique (méthylisobutyl cétone) en présence d'un catalyseur platine bismuth supporté sur charbon (PtBi/C) dans lequel le ratio molaire Bi/Pt est de 1 (M.Kröger et al., Topics in Catalysis, 2000, 13, 237-242.). Ce procédé présente l'inconvénient de nécessiter l'utilisation de solvant organique et également une quantité importante de bismuth. De plus, le rendement de l'étape d'oxydation est faible.

Enfin, il a récemment été proposé (WO2011/043660) un procédé de préparation de FDCA à partir de fructose comprenant la transformation, dans un solvant alcoolique, du fructose en éther de HMF, puis l'oxydation des éthers de HMF formés en FDCA. L'oxydation se fait en milieu acide acétique à 130°C en présence d'un catalyseur soluble comprenant du cobalt et du manganèse, en présence d'acide bromique. Les conditions de mise en oeuvre de l'oxydation sont donc complexes notamment du fait de l'utilisation de bromure et d'acide acétique. De plus, le catalyseur étant soluble, il n'est ainsi pas possible de le recycler. Le rendement en FDCA est d'environ 80%.

Il existe donc un intérêt tout particulier à fournir un procédé de préparation de FDCA à partir de HMF ne nécessitant ni solvant organique, ni base forte, ni composé bromé.

Un objectif de la présente invention est de fournir un procédé de préparation de FDCA par oxydation du HMF par l'air dans l'eau.

Un autre objectif encore de l'invention est de fournir un procédé de préparation de FDCA ne nécessitant pas l'utilisation de solvant organique et de composé bromé.

Un autre objectif de la présente invention est de fournir un procédé de préparation de FDCA permettant d'obtenir de bons rendements, notamment des rendements quantitatifs avec un temps de réaction court.

Un autre objectif de l'invention est de fournir un procédé de préparation de FDCA mettant en oeuvre un catalyseur solide pouvant être facilement recyclé sans nécessité de prétraitement.

D'autres objectifs encore apparaîtront à la lumière de la description de l'invention qui suit.

Les objectifs précités sont remplis par la présente invention qui concerne un procédé de préparation d'acide 2,5-furane dicarboxylique (FDCA) comprenant l'oxydation du 5-hydroxyméthylfurfural (HMF) dans l'eau en présence d'une base faible et d'un catalyseur supporté comprenant du platine et du bismuth, dans lequel le ratio molaire Bi/ Pt dans le catalyseur est compris entre 0,1 à 0,3. Dans le cadre de la présente invention on entend par « base faible » les bases dont le pKb est supérieur à 1,4, de préférence compris entre 3 et 10, par exemple entre 3 et 8 à 25°C en solution diluée. La base faible peut notamment être choisie parmi les carbonates et hydrogénocarbonates de métaux alcalins, notamment sodium et potassium, seuls ou en mélange. De préférence la base est le carbonate de sodium.

L'utilisation d'une base faible permet avantageusement de limiter les réactions secondaires, notamment de décomposition du HMF, réactions de Cannizzaro, qui peuvent se produire en milieux fortement alcalin (base forte). L'utilisation d'une base faible permet également avantageusement d'obtenir le FDCA sous forme de sel ce qui évite son adsorption à la surface du catalyseur et donc l'empoisonnement du catalyseur, tout en maintenant un pH favorable à la réaction.

Dans le cadre de l'invention et sauf indication contraire, l'expression « compris entre xxx et yyy » doit être entendue comme comprenant les bornes xxx et yyy.

L'oxydation peut être mise en oeuvre de toute manière connue de l'homme du métier et notamment sous pression d'air ou d'oxygène, de préférence sous pression d'air. De préférence, la pression d'air ou d'oxygène mise en oeuvre est telle que le rapport molaire O₂/HMF est supérieur à 2.La pression peut notamment être de 20 à 60 bar (2 à 6 MPa) d'air, de préférence de 30 à 50 bar d'air (3 à 5 MPa), par exemple de 40 bar d'air (4 MPa). La pression partielle en oxygène peut être de 4 à 12 bar, de préférence de 6 à 10 bar, par exemple de 8 bar.

Le procédé peut être mené à une température de 70 à 150°C, de préférence de 80 à 120°C, par exemple à 100°C.

Le catalyseur de l'invention est un catalyseur comprenant du platine et du bismuth sur un support. Le catalyseur de l'invention est dénommé ci-après PtBi/support.

De façon avantageuse, la présence de bismuth permet de protéger le platine contre la sur-oxydation. Ainsi et de façon avantageuse, le catalyseur de l'invention peut être réutilisé plusieurs fois sans avoir besoin de lui faire subir une réduction préalable, contrairement au catalyseur monométallique. Cela peut permettre notamment d'augmenter le rendement d'une installation industrielle.

Le support du catalyseur peut être choisi parmi tous les supports connus de l'homme du métier et plus particulièrement parmi les supports stables dans l'eau et de préférence en milieu basique. Le support est de préférence choisi parmi le charbon actif, les carbones, les oxydes métalliques ou de lanthanides stables dans des milieux aqueux, notamment milieux aqueux basiques, par exemple les oxydes de zirconium, de titane, de cérium, ou leur mélange. De préférence, le support est le charbon actif.

Le catalyseur selon l'invention peut être préparé par toute méthode connue de l'homme du métier.

De préférence, le catalyseur est préparé en deux étapes, une première étape de préparation d'un platine supporté et une deuxième étape d'ajout du bismuth, de telles méthodes ont, par exemple, été décrites par T. Mallat, A. Baiker (Chem. Rev. 2004, 104, 3037).

De préférence, la première étape est réalisée par imprégnation en phase liquide du support avec une solution aqueuse de platine, notamment une solution aqueuse de H₂PtCl₆, suivie d'une réduction par une solution de formaldéhyde en milieu basique. Le catalyseur final PtBi/support de l'invention est préparé à partir du platine supporté obtenu à la première étape :
- soit in situ, dans le milieu réactionnel comprenant le platine supporté du procédé d'oxydation du HMF, par ajout d'une solution aqueuse d'un sel de bismuth soluble, notamment nitrate de bismuth Bi(NO₃)₃.5H₂O ;
- soit ex situ par addition au platine supporté d'une solution aqueuse d'un sel de bismuth soluble comprenant un réducteur, par exemple glucose, notamment oxynitrate de bismuth (BiONO₃).

Dans ces deux cas, le catalyseur PtBi/support est obtenu par réaction d'oxydoréduction de surface entre le platine supporté et le sel de bismuth en solution aqueuse.

De préférence, la quantité de platine dans le catalyseur est comprise entre 1% et 10%, de préférence entre 3% et 6% en poids par rapport au poids total du catalyseur supporté.

De préférence dans le catalyseur de l'invention le ratio molaire Bi/Pt est compris entre 0,15 et 0,3. De façon avantageuse ces gammes de ratio molaire Bi/Pt permettent d'obtenir un rendement quantitatif ou quasiment quantitatif, notamment supérieur ou égal à 95%, voire supérieur ou égal à 99%, en un temps de réaction réduit. En effet, du fait de la sensibilité du HMF en solution aqueuse basique il est nécessaire d'avoir un procédé qui permette une conversion totale ou quasiment totale en un temps réduit. Ainsi, de façon avantageuse le procédé est mis en oeuvre pendant 1 h à 10 h, de préférence pendant 2 h à 4 h. Les gammes de ratio molaire Bi/Pt précitées permettent d'obtenir le meilleur compromis entre temps de réaction et rendement en FDCA. En dehors de ces gammes il est nécessaire d'avoir un temps de réaction plus important pour obtenir un rendement en FDCA quantitatif accentuant donc les risques de dégradation du HMF.

Le procédé de la présente invention est de préférence mis en oeuvre avec un ratio molaire base faible/HMF supérieur ou égal à 1, de préférence compris entre 2 et 6, par exemple compris entre 2 et 4.

Le procédé de la présente invention est de préférence mis en oeuvre avec un ratio molaire HMF/Pt compris entre 50 et 400, de préférence entre 100 et 200. De façon avantageuse, la présence de bismuth dans le catalyseur de l'invention, qui permet de protéger le platine de la sur-oxydation, et notamment dans les ratios spécifiés ci-dessus, permet également de limiter la quantité de platine à mettre en oeuvre dans le procédé de l'invention. Le procédé de l'invention s'en trouve ainsi plus économique et plus environnemental par rapport aux procédés de l'état de la technique.

Le procédé de l'invention est mis en oeuvre dans l'eau. Il doit par conséquent être compris que le milieu réactionnel ne comprend pas de solvant organique.

De façon particulièrement préférée, dans le procédé de l'invention, la base faible est le carbonate de calcium ou de potassium et le ratio molaire Bi/Pt est compris entre 0,05 et 2,5, de préférence il est de 0,1 à 0,3, par exemple de 0,15 à 0,3.

Le procédé de la présente invention peut être mis en oeuvre en batch ou en continu. Dans un mode de réalisation avantageux le procédé est mis en oeuvre en continu.

La présente invention va maintenant être décrite à l'aide d'exemples.

### Procédé de préparation des catalyseurs

Les charbons utilisés pour la préparation des catalyseurs sont les charbons L3S et 3SW de la Société CECA.

La méthode employée a été décrite par Dirkx et Van Der Baan (J. M. H. Dirkx et H. S. van Der Baan, Journal of Catalysis 67 (1981) 1-13).

### Catalyseur 5,1%Pt/C(3SW)

Dans un ballon tricol de 250 mL, 10 g de charbon CECA 3SW sont mis en suspension dans 100 mL d'eau ultra pure à température ambiante sous flux d'azote et sous agitation. Une solution aqueuse de 12 mL contenant le précurseur métallique (H₂PtCl₆, 6H₂O) 1,7 g est ajoutée goutte à goutte. L'imprégnation est opérée à température ambiante pendant 5 heures. Puis la suspension est refroidie à une température inférieure à 5°C. La réduction du catalyseur est réalisée en phase liquide par ajout de 50 mL de formaldéhyde aqueux (37% en poids dans l'eau) goutte à goutte pendant 20 min, suivi d'un ajout de 20 mL solution de KOH à 30% précautionneusement de façon à garder la température inférieur à 5°C. On laisse la réduction se poursuivre toute la nuit à température ambiante et toujours sous bullage d'azote. Enfin, la suspension est filtrée et lavée jusqu'à neutralité du filtrat. Le catalyseur récupéré est séché sous flux d'azote à 80°C pendant une journée. Après séchage, le catalyseur est stocké sous atmosphère inerte.

Le catalyseur 3,6%Pt/C (L3S) a été préparé de la même façon sur le charbon L3S.

Les catalyseurs bimétalliques (PtBi/C) sont préparés soit ex-situ à partir du catalyseur monométallique (Pt/C) préparés selon le procédé ci-dessus en déposant le bismuth par réaction d'oxydo-réduction entre la surface de Pt et l'oxyde de bismuth en présence de glucose comme agent réducteur. La réaction est opérée à 40°C sous balayage d'azote et sous agitation magnétique pendant 1 h. Les catalyseurs peuvent être aussi préparés in-situ au cours de la réaction d'oxydation en ajoutant une quantité de sel de bismuth (correspondant au ratio Bi/Pt souhaité).

### Catalyseur 5,1%Pt1%Bi/C(3SW) - ex situ

Dans un ballon tricol de 500 mL équipé d'un barreau aimanté et d'un système de bullage d'azote, 5 g de catalyseur monométallique 5,1%Pt/C(3SW) sont mis en suspension dans 300 mL d'eau ultra pure. La suspension est chauffée jusqu'à atteindre une température de 40°C à l'aide d'une plaque chauffante équipée d'un thermocouple et d'un bain d'eau. Après 20 minutes, on ajoute 186 g de glucose (correspondant à un rapport molaire de glucose/Pt de 790) à l'aide d'un entonnoir à solides, puis on laisse agiter pendant 10 minutes. Ensuite, une solution d'oxynitrate de bismuth (0,06 g de BiONO₃ solubilisé dans 3 mL d'une solution de HCl à 1 M) est ajoutée goutte à goutte. Après environ 1 heure d'agitation, le chauffage est arrêté et le système refroidi. La suspension est neutralisée par ajout d'une solution de NaOH à 0,2 M jusqu'à atteindre un pH supérieur à 9. Enfin, la suspension est filtrée et lavée jusqu'à neutralité du filtrat. Le catalyseur récupéré est séché sous flux d'azote à 80°C pendant une journée. Après séchage, le catalyseur est stocké sous atmosphère inerte.

Les autres catalyseurs avec des ratios Bi/Pt différents ont été préparés de la même façon en ajustant la quantité de sel de bismuth selon le tableau suivant pour 1 g de catalyseur bimétallique préparé.

| Catalyseur | Pt/C mis en oeuvre dans le procédé | Quantité de BiONO₃ [mg] | Ratio Bi/Pt |
|---|---|---|---|
| 5%Pt0,4%Bi/C(3SW) | 5,1%Pt/C(3SW) | 6 | 0,07 |
| 5%Pt0,7%Bi/C(3SW) | 5,1%Pt/C(3SW) | 8 | 0,13 |
| 5,1%Pt1%Bi/C(3SW) | 5,1%Pt/C(3SW) | 11 | 0,20 |
| 3,6%Pt0,9%Bi/C(L3S) | 3,6%Pt/C(L3S) | 10 | 0,25 |
| 4,5%Pt1,65%Bi/C(3SW) | 5,1%Pt/C(3SW) | 20 | 0,36 |
| 3,6%Pt1,9%Bi/C(L3S) | 3,6%Pt/C(L3S) | 19 | 0,52 |
| 3,5%Pt3,4%Bi/C(L3S) | 3,6%Pt/C(L3S) | 39 | 0,96 |

### Catalyseur 3,6%Pt0,68%Bi/C(L3S) - in situ

On met en suspension dans le réacteur 15 mg de sel de bismuth (Bi(NO₃)₃, 5H₂O), correspondant à un ratio molaire Bi/Pt de 0,2, dans 150 mL d'une solution à 0,2 M de Na₂CO₃. 0,816 g du catalyseur monométallique 3,6%Pt/C(L3S) sont ajoutés. Après fermeture du réacteur, la suspension est mise sous agitation et sous atmosphère inerte pendant 1 heure. 2 g d'HMF sont ensuite ajoutés et le test catalytique est directement démarré en suivant le protocole standard décrit ci-dessous. A la fin de la réaction le catalyseur est récupéré et analysé.

### Conditions expérimentales

Le HMF est fourni par la société Interchim avec une pureté de 95%.

Le procédé de l'invention est mis en oeuvre en réacteur discontinu sous pression dans un autoclave de 300 mL équipé d'un mobile d'agitation à effet gazeux à entraînement magnétique. Le chauffage est assuré par un collier chauffant relié à un régulateur PID (proportionnel intégral dérivé). Une vanne de prélèvement permet de prélever un échantillon du milieu réactionnel via un tube plongeant, ce qui permet de suivre l'évolution de la réaction au cours du temps. Les prélèvements sont analysés par chromatographie HPLC avec deux détecteurs RID (Détecteur à Indice de Réfraction) et PDA (détecteur à barrette de diodes) (colonne ICE-Coragel 107H, éluant 10 mM H₂SO₄). Le Carbone Organique Total (COT) en solution est également analysé à l'aide d'un analyseur de COT et la valeur mesurée est comparée au bilan molaire (BM) calculé par HPLC.

Le réacteur est chargé avec 150 mL d'une solution aqueuse 100 mM de HMF (2 g), une masse de catalyseur correspondant à un rapport molaire HMF/Pt de 100 et la quantité de base souhaitée sous forme de NaOH (exemple comparatif), NaHCO₃, KHCO₃, Na₂CO₃ ou K₂CO₃ exprimée en rapport molaire base/HMF. L'air est introduit à la pression de 40 bar, le réacteur est chauffé jusqu'à 100°C.

### Exemple 1 (comparatif) : Catalyseurs monométalliques en présence de NaHCO₃

La réaction décrite ci-dessus a été menée en présence de deux catalyseurs Pt supporté sur deux types de charbon (CECA L3S et 3SW) et d'un ratio molaire NaHCO₃/HMF de 4. Les résultats sont représentés dans les tableaux 1 et 2 ci-dessous.

**Tableau 1**

| **3,6%Pt/C(L3S)** | | | | | | | | |
|---|---|---|---|---|---|---|---|---|
| **Durée [h]** | **pH** | **HMF [mM]** | **HMFCA [mM]** | **DFF [mM]** | **FFCA [mM]** | **FDCA [mM]** | **BM [mM]** | **COT [mM]** |
| 0 | 8,64 | 99 | 0 | 0 | 0 | 0 | 99 | 100 |
| 0,25 | 8,57 | 52 | 5 | 3 | 31 | 1 | 91 | |
| 0,5 | 8,39 | 8 | 7 | 0 | 68 | 8 | 91 | |
| 1 | 8,25 | 1 | 5 | 0 | 68 | 20 | 94 | |
| 2 | 8,15 | 0 | 2 | 0 | 53 | 39 | 95 | 94 |
| 3 | 8,22 | 0 | 0 | 0 | 43 | 50 | 94 | |
| 4 | 8,25 | 0 | 0 | 0 | 34 | 61 | 95 | |
| 5 | 8,19 | 0 | 0 | 0 | 29 | 66 | 95 | |
| 6 | 8,07 | 0 | 0 | 0 | 23 | 72 | 95 | 96 |
| 8 | 8,03 | 0 | 0 | 0 | 14 | 81 | 95 | |
| 10 | 7,68 | 0 | 0 | 0 | 8 | 88 | 96 | |
| 12 | 7,52 | 0 | 0 | 0 | 5 | 90 | 96 | 94 |

**Tableau 2**

| **5,1%Pt/C(3SW)** | | | | | | | | |
|---|---|---|---|---|---|---|---|---|
| **Durée [h]** | **pH** | **HMF [mM]** | **HMFCA [mM]** | **DFF [mM]** | **FFCA [mM]** | **FDCA [mM]** | **BM [mM]** | **COT [mM]** |
| 0 | 8,61 | 100 | 0 | 0 | 0 | 0 | 100 | 94 |
| 0,25 | 8,51 | 42 | 5 | 3 | 40 | 2 | 92 | |
| 0,5 | 8,51 | 16 | 8 | 2 | 62 | 6 | 94 | |
| 1 | 8,42 | 1 | 7 | 0 | 71 | 18 | 96 | |
| 2 | 8,35 | 0 | 4 | 0 | 59 | 34 | 97 | 97 |
| 3 | 8,33 | 0 | 2 | 0 | 48 | 47 | 97 | |
| 4 | 8,31 | 0 | 0 | 0 | 41 | 55 | 96 | |
| 5 | 8,25 | 0 | 0 | 0 | 34 | 62 | 96 | |
| 6 | 8,33 | 0 | 0 | 0 | 28 | 69 | 97 | 96 |
| 8 | 8,43 | 0 | 0 | 0 | 21 | 77 | 97 | |
| 10 | 8,24 | 0 | 0 | 0 | 14 | 83 | 97 | |
| 12 | 8,09 | 0 | 0 | 0 | 9 | 89 | 98 | 96 |

Les résultats montrent que la conversion de l'HMF est rapide en moins de 1 h pour donner comme intermédiaires le 2,5-diformylfurane (DFF) et l'acide 5-hydroxyméthyl-2-furane carboxylique (HMFCA) qui sont facilement oxydés en acide 2-formyl-5-furane carboxylique (FFCA). L'oxydation de la fonction aldéhyde du FFCA pour donner le FDCA est beaucoup plus lente. Après 24 h de réaction, la conversion de FFCA est complète et le rendement en FDCA est quantitatif. La nature du support charbon (pH acide pour L3S et basique pour 3SW, selon les données du fournisseur) n'a pas d'influence sur l'activité des catalyseurs.

La réaction a été menée avec NaOH comme base à la place de NaHCO₃. Dans ces conditions l'HMF s'est dégradé très rapidement.

### Exemple 2 (comparatif) : Influence de la nature de la base en présence d'un catalyseur monométallique Pt/C

La réaction décrite ci-dessus a été menée en présence d'un catalyseur 5,1%Pt/C(3SW) et de deux bases faibles, NaHCO₃ et K₂CO₃ avec des ratios base/HMF respectivement de 4 et 2.

Les résultats sont regroupés dans les tableaux 3 et 4 ci-dessous.

**Tableau 3**

| **ratio molaire NaHCO₃/HMF de 4** | | | | | | |
|---|---|---|---|---|---|---|
| **Durée [h]** | **HMF [mM]** | **%C HMF** | **FFCA [mM]** | **%R FFCA** | **FDCA [mM]** | **%R FDCA** |
| 0 | 100 | 0 | 0 | 0 | 0 | 0 |
| 0,25 | 42 | 58 | 40 | 40 | 2 | 2 |
| 0,5 | 16 | 84 | 62 | 62 | 6 | 6 |
| 1 | 1 | 99 | 71 | 71 | 18 | 18 |
| 2 | 0 | 100 | 59 | 59 | 34 | 34 |
| 3 | 0 | 100 | 48 | 48 | 47 | 47 |
| 4 | 0 | 100 | 41 | 41 | 55 | 55 |
| 5 | 0 | 100 | 34 | 34 | 62 | 62 |
| 6 | 0 | 100 | 28 | 28 | 69 | 69 |
| 8 | 0 | 100 | 21 | 21 | 77 | 77 |
| 10 | 0 | 100 | 14 | 14 | 83 | 83 |
| 12 | 0 | 100 | 9 | 9 | 89 | 89 |

| | | | | | | |
|---|---|---|---|---|---|---|
| (%RFFCA=rendement (%) en FFCA, %RFDCA= rendement (%) en FDCA %C = conversion (%) en H MF) | | | | | | |

**Tableau 4**

| **ratio molaire K₂CO₃/HMF de 2** | | | | | | |
|---|---|---|---|---|---|---|
| **Durée [h]** | **HMF [mM]** | **%C HMF** | **FFCA [mM]** | **%R FFCA** | **FDCA [mM]** | **%R FDCA** |
| 0 | 96 | 0 | 0 | 0 | 0 | 0 |
| 0,25 | 25 | 73 | 38 | 39 | 13 | 13 |
| 0,5 | 0 | 99 | 45 | 47 | 32 | 34 |
| 1 | 0 | 100 | 34 | 35 | 49 | 51 |
| 2 | 0 | 100 | 23 | 24 | 63 | 66 |
| 3 | 0 | 100 | 18 | 18 | 71 | 73 |
| 4 | 0 | 100 | 13 | 14 | 78 | 81 |
| 6 | 0 | 100 | 9 | 9 | 83 | 86 |
| 8 | 0 | 100 | 6 | 6 | 87 | 90 |
| 10 | 0 | 100 | 4 | 4 | 89 | 93 |
| 12 | 0 | 100 | 3 | 3 | 91 | 94 |

| | | | | | | |
|---|---|---|---|---|---|---|
| (%RFFCA=rendement (%) en FFCA, %RFDCA= rendement (%) en FDCA %C = conversion (%) en H MF) | | | | | | |

Les résultats montrent que l'utilisation d'une base faible plus forte que NaHCO₃ (K₂CO₃ ou Na₂CO₃) permet d'améliorer les vitesses d'oxydation. Ainsi, en présence du catalyseur 5,1%Pt/C(3SW), le temps de réaction nécessaire pour atteindre un rendement quantitatif est d'environ 14 heures.

### Exemple 3 (comparatif) : Etude du recyclage du catalyseur 3.6%Pt/C(L3S)

Le catalyseur 3.6%Pt/C(L3S) a été utilisé dans trois réactions d'oxydation du HMF dans les conditions décrites ci-dessus. Il a été mis en oeuvre dans une première réaction d'oxydation du HMF (1^{er} cycle) en présence de 4 équivalents de NaHCO₃ (par rapport au HMF). Après 24 heures de réaction, le catalyseur a été récupéré par filtration, ensuite lavé, séché à l'étuve à 100°C à l'air, puis réduit sous flux d'hydrogène à 350°C pendant 2 heures. Après cette étape de traitement, le catalyseur a été mis en oeuvre de nouveau dans une deuxième réaction d'oxydation du HMF (2^{ème} cycle) dans des conditions identiques. Il a ensuite été lavé à l'eau et séché à l'étuve à 100°C à l'air. Le catalyseur a ensuite directement (sans étape de réduction préalable) été mis en oeuvre dans une troisième réaction d'oxydation du HMF (3^{ème} cycle) dans des conditions identiques.

Les résultats obtenus sont regroupés dans le tableau 5 pour le 1^{er} cycle, dans le tableau 6 pour le 2^{ème} cycle et dans le tableau 7 pour le 3^{ème} cycle.

**Tableau 5**

| **1^{er} cycle** | | | | | | | | |
|---|---|---|---|---|---|---|---|---|
| **Durée [h]** | **pH** | **HMF [mM]** | **HMFCA [mM]** | **DFF [mM]** | **FFCA [mM]** | **FDCA [mM]** | **BM [mM]** | **COT [mM]** |
| 0 | 8,58 | 95 | 0 | 0 | 0 | 0 | 95 | 96 |
| 0,5 | 8,41 | 27 | 6 | 1 | 52 | 6 | 91 | |
| 1 | 8,28 | 1 | 5 | 0 | 67 | 23 | 95 | |
| 2 | 8,29 | 0 | 1 | 0 | 44 | 51 | 95 | |
| 2,7 | 8,09 | 0 | 0 | 0 | 33 | 61 | 94 | |
| 4 | 7,69 | 0 | 0 | 0 | 23 | 72 | 95 | 97 |
| 5,8 | 7,98 | 0 | 0 | 0 | 16 | 79 | 95 | |
| 8 | 7,94 | 0 | 0 | 0 | 8 | 87 | 94 | |
| 10 | 7,9 | 0 | 0 | 0 | 4 | 91 | 94 | 97 |
| 24 | 7,48 | 0 | 0 | 0 | 0 | 95 | 95 | 98 |

**Tableau 6**

| 2^{ème} cycle | | | | | | | | |
|---|---|---|---|---|---|---|---|---|
| **Durée [h]** | **pH** | **HMF [mM]** | **HMFCA [mM]** | **DFF [mM]** | **FFCA [mM]** | **FDCA [mM]** | **BM [mM]** | **COT [mM]** |
| 0 | 8,55 | 99 | 0 | 0 | 0 | 0 | 99 | 100 |
| 0,5 | 8,09 | 15 | 0 | 0 | 62 | 16 | 94 | 96 |
| 1 | 8,11 | 0 | 0 | 0 | 63 | 35 | 99 | |
| 2 | 7,83 | 0 | 0 | 0 | 41 | 58 | 99 | |
| 3 | 7,88 | 0 | 0 | 0 | 27 | 72 | 99 | |
| 4 | 7,74 | 0 | 0 | 0 | 18 | 81 | 99 | |
| 6 | 7,78 | 0 | 0 | 0 | 8 | 90 | 99 | |
| 8 | 7,69 | 0 | 0 | 0 | 3 | 97 | 100 | |
| 10 | 7,65 | 0 | 0 | 0 | 1 | 99 | 100 | 101 |
| 24 | 7,48 | 0 | 0 | 0 | 0 | 99 | 99 | 97 |

**Tableau 7**

| **3^{ème} cycle** | | | | | | | | |
|---|---|---|---|---|---|---|---|---|
| **Durée [h]** | **pH** | **HMF [mM]** | **HMFCA [mM]** | **DFF [mM]** | **FFCA [mM]** | **FDCA [mM]** | **BM [mM]** | **COT [mM]** |
| 0 | 8,45 | 99 | 0 | 0 | 0 | 0 | 99 | 99 |
| 0,5 | 8,25 | 56 | 1 | 4 | 28 | 1 | 90 | 95 |
| 1 | 8,07 | 29 | 2 | 3 | 52 | 3 | 89 | |
| 2 | 7,95 | 3 | 2 | 0 | 75 | 13 | 93 | |
| 3 | 7,86 | 0 | 3 | 0 | 69 | 22 | 94 | |
| 4 | 7,86 | 0 | 2 | 0 | 62 | 32 | 95 | |
| 6 | 7,75 | 0 | 1 | 0 | 50 | 47 | 97 | |
| 8 | 7,79 | 0 | 0 | 0 | 38 | 60 | 98 | |
| 10 | 7,52 | 0 | 0 | 0 | 28 | 70 | 98 | 101 |
| 24 | 7,48 | 0 | 0 | 0 | 2 | 97 | 99 | 102 |

Les résultats montrent que pour les 1^{er} et 2^{ème} cycles le rendement est quantitatif après 10 heures de réaction. En revanche, une perte d'activité sensible est observée lors du 3^{ème} cycle, avec un rendement de 70% seulement après 10 h de réaction. Le catalyseur monométallique Pt/C nécessite une étape de réduction avant sa réutilisation.

### Exemple 4 (selon l'invention) : Influence de la modification des catalyseurs monométalliques Pt/C par du bismuth

Les essais ont été menés avec les catalyseurs bimétalliques PtBi/C préparés selon les protocoles mentionnés ci-dessus avec des rapports molaires Bi/Pt variables entre 0,07 et 1.

Les tests en présence de NaHCO₃ ont été mis en oeuvre avec un ratio molaire NaHCO₃/HMF de 4 à une température de 100°C et une pression d'air de 40 bar.

Les résultats sont regroupés dans les tableaux 8, 9, et 10

**Tableau 8**

| **Catalyseur 5,1%Pt-1%Bi/C(3SW) (rapport molaire Bi/Pt=0,2)** | | | | | | | | |
|---|---|---|---|---|---|---|---|---|
| **Durée [h]** | **pH** | **HMF [mM]** | **HMFCA [mM]** | **DFF [mM]** | **FFCA [mM]** | **FDCA [mM]** | **BM [mM]** | **COT [mM]** |
| 0 | 8,66 | 101 | 0 | 0 | 0 | 0 | 101 | 100 |
| 0,25 | 8,49 | 31 | 8 | 2 | 49 | 5 | 95 | 99,3 |
| 0,5 | 8,32 | 3 | 8 | 0 | 67 | 20 | 97 | |
| 1 | 8,37 | 0 | 4 | 0 | 52 | 43 | 98 | |
| 2 | 8,29 | 0 | 1 | 0 | 28 | 70 | 98 | |
| 3 | 8,25 | 0 | 0 | 0 | 14 | 85 | 99 | |
| 4 | 8,17 | 0 | 0 | 0 | 7 | 92 | 99 | |
| 5 | 8,17 | 0 | 0 | 0 | 3 | 97 | 99 | |
| 6 | 8,1 | 0 | 0 | 0 | 1 | 98 | 100 | 100,7 |
| 8 | 8,24 | 0 | 0 | 0 | 0 | 99 | 100 | |
| 10 | 8,12 | 0 | 0 | 0 | 0 | 100 | 100 | |
| 12 | 7,74 | 0 | 0 | 0 | 0 | 100 | 100 | 101,1 |

**Tableau 9**

| **3,6%Pt-0,9%Bi/C(L3S) - Bi/Pt=0,25** | | | | | | | | |
|---|---|---|---|---|---|---|---|---|
| **Durée [h]** | **pH** | **HMF [mM]** | **HMFCA [mM]** | **DFF [mM]** | **FFCA [mM]** | **FDCA [mM]** | **BM [mM]** | **COT [mM]** |
| 0 | 8,61 | 95 | 0 | 0 | 0 | 0 | 95 | 92 |
| 0,16 | 8,37 | 55 | 5 | 3 | 29 | 1 | 94 | |
| 0,33 | 8,18 | 13 | 8 | 1 | 64 | 8 | 95 | 94,7 |
| 0,5 | 8,13 | 1 | 7 | 0 | 70 | 18 | 96 | |
| 0,75 | 8,15 | 0 | 5 | 0 | 63 | 29 | 97 | |
| 1 | 8,02 | 0 | 4 | 0 | 56 | 38 | 97 | 96,57 |
| 2 | 7,89 | 0 | 1 | 0 | 38 | 60 | 100 | |
| 3 | 7,85 | 0 | 0 | 0 | 19 | 80 | 99 | |
| 4 | 7,68 | 0 | 0 | 0 | 10 | 89 | 99 | 99,01 |
| 6 | 7,64 | 0 | 0 | 0 | 3 | 96 | 100 | |
| 8 | 7,70 | 0 | 0 | 0 | 1 | 99 | 100 | |
| 10 | 7,39 | 0 | 0 | 0 | 0 | 100 | 100 | 100 |
| 12 | 7,43 | 0 | 0 | 0 | 0 | 101 | 101 | 101 |

**Tableau 10**

| **4,5%Pt-1,65%Bi/C(3SW) - Bi/Pt=0,36** | | | | | | | | |
|---|---|---|---|---|---|---|---|---|
| Durée [h] | **pH** | **HMF [mM]** | **HMFCA [mM]** | **DFF [mM]** | **FFCA [mM]** | **FDCA [mM]** | **BM [mM]** | **COT [mM]** |
| 0 | 8,40 | 97 | 0 | 0 | 0 | 0 | 97 | 96 |
| 0,25 | 8,27 | 50 | 6 | 3 | 32 | 2 | 93 | |
| 0,5 | 8,22 | 16 | 9 | 2 | 61 | 7 | 94 | |
| 1 | 8,36 | 0 | 7 | 0 | 68 | 19 | 94 | |
| 2 | 7,83 | 0 | 4 | 0 | 53 | 40 | 96 | 97 |
| 4 | 7,66 | 0 | 0 | 0 | 31 | 66 | 96 | |
| 6 | 7,66 | 0 | 0 | 0 | 16 | 81 | 97 | |
| 8 | 7,58 | 0 | 0 | 0 | 8 | 88 | 96 | 99 |
| 10 | 7,72 | 0 | 0 | 0 | 3 | 94 | 97 | |
| 12 | 7,39 | 0 | 0 | 0 | 0 | 96 | 96 | 97 |

La réaction en présence du catalyseur bimétallique 5.1%Pt-1%Bi/C(3SW) (rapport Bi/Pt=0,2) est très nettement accélérée par rapport à celle observée en présence de catalyseur monométallique 5,1%Pt/C(3SW) (voir exemple comparatif 2). Les conversions de HMF et FFCA sont totales en moins de 6 h à comparer à plus de 12 h pour le catalyseur monométallique Pt/C (exemples comparatifs 1 et 2).
Les résultats montrent que l'augmentation du rapport Bi/Pt par exemple à 0,36 ralentit la réaction.
De la même façon, la modification du catalyseur 3,6%Pt/C(L3S) par du bismuth accélère fortement les vitesses de réaction (voir exemple comparatif 1).

L'influence du rapport Bi/Pt a été aussi étudiée en présence de Na₂CO₃ (Na₂CO₃/HMF=2). La même tendance a été observée en comparant une série de catalyseurs bimétalliques avec des rapports molaires qui varient entre 0,07 et 1. Les résultats sont regroupés dans les tableaux 11 à 15.

**Tableau 11**

| **5%Pt0,4%Bi/C(3SW), Bi/Pt=0,07** | | | | | | | | |
|---|---|---|---|---|---|---|---|---|
| Durée [h] | **pH** | **HMF [mM]** | **HMFCA [mM]** | **DFF [mM]** | **FFCA [mM]** | **FDCA [mM]** | **BM [mM]** | **COT [mM]** |
| 0 | 10,59 | 98 | 0 | 0 | 0 | 0 | 98 | 97 |
| 0,25 | 9,44 | 11 | 11 | 0 | 54 | 15 | 91 | |
| 0,5 | 9,06 | 0 | 8 | 0 | 50 | 38 | 96 | 95 |
| 1 | 8,83 | 0 | 3 | 0 | 32 | 61 | 96 | |
| 2 | 8,66 | 0 | 1 | 0 | 16 | 79 | 96 | 95 |
| 3 | 8,44 | 0 | 0 | 0 | 10 | 85 | 95 | |
| 4 | 8,44 | 0 | 0 | 0 | 6 | 89 | 95 | 96 |
| 5 | 8,33 | 0 | 0 | 0 | 3 | 93 | 96 | |
| 6 | 8,41 | 0 | 0 | 0 | 2 | 94 | 96 | 96 |

**Tableau 12**

| **5%Pt0,7%Bi/C(3SW), Bi/Pt=0,13** | | | | | | | | |
|---|---|---|---|---|---|---|---|---|
| **Durée [h]** | **pH** | **HMF [mM]** | **HMFCA [mM]** | **DFF [mM]** | **FFCA [mM]** | **FDCA [mM]** | **BM [mM]** | **COT [mM]** |
| 0 | 10,44 | 98 | 0 | 0 | 0 | 0 | 98 | 100 |
| 0,25 | 9,39 | 13 | 10 | 0 | 52 | 20 | 94 | |
| 0,5 | 8,90 | 0 | 6 | 0 | 40 | 49 | 95 | 95 |
| 1 | 8,70 | 0 | 1 | 0 | 21 | 75 | 97 | |
| 2 | 8,49 | 0 | 0 | 0 | 9 | 87 | 96 | 97 |
| 3 | 8,49 | 0 | 0 | 0 | 3 | 93 | 96 | |
| 4 | 8,47 | 0 | 0 | 0 | 1 | 96 | 97 | 99 |
| 5 | 8,61 | 0 | 0 | 0 | 1 | 96 | 97 | |
| 6 | 8,53 | 0 | 0 | 0 | 0 | 97 | 97 | 98 |

**Tableau 13**

| **5,1%Pt1%Bi/C(3SW), Bi/Pt=0,2** | | | | | | | | |
|---|---|---|---|---|---|---|---|---|
| **Durée [h]** | **pH** | **HMF [mM]** | **HMFCA [mM]** | **DFF [mM]** | **FFCA [mM]** | **FDCA [mM]** | **BM [mM]** | **COT [mM]** |
| 0 | 11,05 | 96 | 0 | 0 | 0 | 0 | 96 | 96 |
| 0,1 | 10,12 | 45 | 5 | 0 | 38 | 3 | 91 | 91 |
| 0,17 | 9,51 | 8 | 10 | 0 | 59 | 17 | 95 | 95 |
| 0,33 | 9,10 | 0 | 6 | 0 | 42 | 47 | 95 | |
| 0,5 | 8,86 | 0 | 3 | 0 | 29 | 63 | 95 | 97 |
| 1 | 8,58 | 0 | 1 | 0 | 16 | 79 | 96 | 97 |
| 2 | 8,34 | 0 | 0 | 0 | 4 | 91 | 95 | |
| 3 | 8,34 | 0 | 0 | 0 | 0 | 95 | 95 | 98 |
| 4 | 8,29 | 0 | 0 | 0 | 0 | 95 | 95 | |
| 5 | 8,25 | 0 | 0 | 0 | 0 | 96 | 96 | 98 |
| 6 | 8,24 | 0 | 0 | 0 | 0 | 96 | 96 | 97 |

**Tableau 14**

| **3,6%Pt1,9%Bi/C(L3S), Bi/Pt=0,52** | | | | | | | | |
|---|---|---|---|---|---|---|---|---|
| **Durée [h]** | **pH** | **HMF [mM]** | **HMFCA [mM]** | **DFF [mM]** | **FFCA [mM]** | **FDCA [mM]** | **BM [mM]** | **COT [mM]** |
| 0 | 10,46 | 98 | 0 | 0 | 0 | 0 | 98 | 100 |
| 0,25 | 9,55 | 21 | 11 | 0 | 47 | 14 | 93 | |
| 0,5 | 9,09 | 0 | 7 | 0 | 45 | 42 | 94 | 94 |
| 1 | 8,7 | 0 | 4 | 0 | 28 | 64 | 96 | |
| 2 | 8,36 | 0 | 1 | 0 | 12 | 84 | 97 | 97 |
| 3 | 8,43 | 0 | 0 | 0 | 5 | 91 | 96 | |
| 4 | 8,29 | 0 | 0 | 0 | 3 | 94 | 97 | 97 |
| 5 | 8,36 | 0 | 0 | 0 | 1 | 95 | 96 | |
| 6 | 8,29 | 0 | 0 | 0 | 1 | 97 | 98 | 99 |

**Tableau 15**

| **3,5%Pt3,4%Bi/C(L3S), Bi/Pt=0,96** | | | | | | | | |
|---|---|---|---|---|---|---|---|---|
| **Durée [h]** | **pH** | **HMF [mM]** | **HMFCA [mM]** | **DFF [mM]** | **FFCA [mM]** | **FDCA [mM]** | **BM [mM]** | **COT [mM]** |
| 0 | 10,50 | 97 | 0 | 0 | 0 | 0 | 97 | 100 |
| 0,25 | 9,60 | 17 | 11 | 1 | 57 | 7 | 93 | |
| 0,5 | 9,32 | 0 | 11 | 0 | 62 | 22 | 94 | 97 |
| 1 | 9,00 | 0 | 7 | 0 | 46 | 43 | 96 | |
| 2 | 8,72 | 0 | 3 | 0 | 27 | 67 | 97 | 99 |
| 3 | 8,37 | 0 | 1 | 0 | 15 | 79 | 96 | |
| 4 | 8,33 | 0 | 1 | 0 | 10 | 86 | 96 | |
| 5 | 8,30 | 0 | 0 | 0 | 6 | 89 | 95 | |
| 6 | 8,38 | 0 | 0 | 0 | 4 | 92 | 95 | 97 |

Il existe ainsi une gamme optimale du ratio Bi/Pt dans le catalyseur permettant d'obtenir un rendement quantitatif en FDCA en un temps réduit.

### Exemple 5 (selon l'invention) : Influence de la nature et de la quantité de base

La nature de la base utilisée et la quantité de base ajoutée ont également été étudiées.

Les résultats sont représentés :
- dans les tableaux 16 et 17 pour le catalyseur 5.1%Pt-1%Bi/C(3SW) (Bi/Pt = 0,2) en présence de 4 équivalents de NaHCO₃ ou KHCO₃ par rapport au HMF ;
- dans les tableaux 18, 19 et 20 pour le catalyseur 5.1%Pt-1%Bi/C(3SW) (Bi/Pt = 0,2) en présence de 2 équivalents de Na₂CO₃ ou K₂CO₃ ou 4 équivalents de Na₂CO₃ par rapport au HMF.

**Tableau 16**

| **5,1%Pt-1%Bi/C(3SW) - Bi/Pt=0,2 - 4 éq NaHCO₃** | | | | | | | | |
|---|---|---|---|---|---|---|---|---|
| **Durée [h]** | **pH** | **HMF [mM]** | **HMFCA [mM]** | **DFF [mM]** | **FFCA [mM]** | **FDCA [mM]** | **BM [mM]** | **COT [mM]** |
| 0 | 8,66 | 101 | 0 | 0 | 0 | 0 | 101 | 100 |
| 0,25 | 8,49 | 31 | 8 | 2 | 49 | 5 | 95 | 99 |
| 0,5 | 8,32 | 3 | 8 | 0 | 67 | 20 | 97 | |
| 1 | 8,37 | 0 | 4 | 0 | 52 | 43 | 98 | |
| 2 | 8,29 | 0 | 1 | 0 | 28 | 70 | 98 | |
| 3 | 8,25 | 0 | 0 | 0 | 14 | 85 | 99 | |
| 4 | 8,17 | 0 | 0 | 0 | 7 | 92 | 99 | |
| 5 | 8,17 | 0 | 0 | 0 | 3 | 97 | 99 | |
| 6 | 8,14 | 0 | 0 | 0 | 1 | 98 | 100 | 101 |
| 8 | 8,24 | 0 | 0 | 0 | 0 | 99 | 100 | |
| 10 | 8,12 | 0 | 0 | 0 | 0 | 100 | 100 | |
| 12 | 7,74 | 0 | 0 | 0 | 0 | 100 | 100 | 101 |

**Tableau 17**

| **5,1%Pt-1%Bi/C(3SW) - Bi/Pt=0,2 - 4 éq KHCO₃** | | | | | | | | |
|---|---|---|---|---|---|---|---|---|
| **Durée [h]** | **pH** | **HMF [mM]** | **HMFCA [mM]** | **DFF [mM]** | **FFCA [mM]** | **FDCA [mM]** | **BM [mM]** | **COT [mM]** |
| 0 | 8,50 | 98 | 0 | 0 | 0 | 0 | 98 | 96 |
| 0,25 | 8,42 | 28 | 8 | 2 | 48 | 6 | 92 | |
| 0,5 | 8,15 | 3 | 8 | 0 | 64 | 20 | 94 | |
| 1 | 8,18 | 0 | 4 | 0 | 48 | 43 | 95 | |
| 2 | 8,35 | 0 | 1 | 0 | 29 | 66 | 96 | 95 |
| 3 | 8,12 | 0 | 0 | 0 | 15 | 80 | 95 | |
| 4 | 8,13 | 0 | 0 | 0 | 7 | 89 | 96 | 96 |
| 5 | 7,87 | 0 | 0 | 0 | 3 | 93 | 96 | |
| 6 | 7,78 | 0 | 0 | 0 | 1 | 95 | 97 | |
| 8 | 7,60 | 0 | 0 | 0 | 0 | 97 | 97 | 98 |
| 10 | 7,50 | 0 | 0 | 0 | 0 | 97 | 97 | 98 |
| 12 | 7,50 | 0 | 0 | 0 | 0 | 97 | 97 | 98 |

**Tableau 18**

| **5,1%Pt-1%Bi/C(3SW) - Bi/Pt=0,2 - 2 éq Na₂CO₃** | | | | | | | | |
|---|---|---|---|---|---|---|---|---|
| **Durée [h]** | **pH** | **HMF [mM]** | **HMFCA [mM]** | **DFF [mM]** | **FFCA [mM]** | **FDCA [mM]** | **BM [mM]** | **COT [mM]** |
| 0 | 11,05 | 96 | 0 | 0 | 0,0 | 0,0 | 96 | 96 |
| 0,1 | 10,12 | 45 | 5 | 0 | 37,9 | 3,2 | 91 | 91 |
| 0,17 | 9,51 | 8 | 10 | 0 | 59,2 | 17,4 | 95 | 95 |
| 0,33 | 9,10 | 0 | 6 | 0 | 42,5 | 46,6 | 95 | |
| 0,5 | 8,86 | 0 | 3 | 0 | 28,6 | 63,1 | 95 | 97 |
| 1 | 8,58 | 0 | 0 | 0 | 16,4 | 79,2 | 96 | 97 |
| 2 | 8,34 | 0 | 0 | 0 | 4,0 | 90,8 | 95 | |
| 3 | 8,34 | 0 | 0 | 0 | 0,0 | 95,0 | 95 | 98 |
| 4 | 8,29 | 0 | 0 | 0 | 0,0 | 95,3 | 95 | |
| 5 | 8,25 | 0 | 0 | 0 | 0,0 | 95,8 | 96 | 98 |
| 6 | 8,24 | 0 | 0 | 0 | 0,0 | 95,7 | 96 | 97 |

**Tableau 19**

| **5,1%Pt-1%Bi/C(3SW) - Bi/Pt=0,2 - 2 éq K₂CO₃** | | | | | | | | |
|---|---|---|---|---|---|---|---|---|
| **Durée [h]** | **pH** | **HMF [mM]** | **HMFCA [mM]** | **DFF [mM]** | **FFCA [mM]** | **FDCA [mM]** | **BM [mM]** | **COT [mM]** |
| 0 | 10,99 | 98 | 0 | 0 | 0 | 0 | 86 | 85 |
| 0,25 | 9,66 | 23 | 8 | 0 | 29 | 33 | 93 | * |
| 0,5 | 8,88 | 0,0 | 4 | 0 | 21 | 72 | 96 | * |
| 1 | 8,40 | 0,0 | 0 | 0 | 8 | 88 | 96 | * |
| 2 | 8,29 | 0,0 | 0 | 0 | 1 | 95 | 97 | 98 |
| 3 | 8,33 | 0,0 | 0 | 0 | 0 | 96 | 96 | * |
| 4,5 | 8,23 | 0,0 | 0 | 0 | 0 | 97 | 97 | 97 |
| 6 | 8,19 | 0,0 | 0 | 0 | 0 | 97 | 97 | 97 |

**Tableau 20**

| **5,1%Pt-1%Bi/C(3SW) - Bi/Pt=0,2 - 4 éq Na₂CO₃** | | | | | | | | |
|---|---|---|---|---|---|---|---|---|
| **Durée [h]** | **pH** | **HMF [mM]** | **HMFCA [mM]** | **DFF [mM]** | **FFCA [mM]** | **FDCA [mM]** | **BM [mM]** | **COT [mM]** |
| 0 | 10,66 | 97 | 0 | 0 | 0 | 0 | 97 | 98 |
| 0,25 | 9,76 | 18 | 7 | 0 | 23 | 44 | 92 | * |
| 0,5 | 9,50 | 0 | 2 | 0 | 10 | 82 | 95 | * |
| 1 | 9,44 | 0 | 0 | 0 | 2 | 92 | 94 | * |
| 2 | 9,40 | 0 | 0 | 0 | 0 | 95 | 95 | 94 |
| 3 | 9,36 | 0 | 0 | 0 | 0 | 95 | 95 | * |
| 4 | 9,33 | 0 | 0 | 0 | 0 | 96 | 96 | 95 |
| 5 | 9,31 | 0 | 0 | 0 | 0 | 95 | 95 | * |
| 6 | 9,30 | 0 | 0 | 0 | 0 | 96 | 96 | 95 |
| 8 | 9,28 | 0 | 0 | 0 | 0 | 96 | 96 | * |
| 12 | 9,26 | 0 | 0 | 0 | 0 | 97 | 97 | 95 |

Les résultats montrent que la nature du cation n'a pas d'influence mais que le remplacement de 4 équivalents des bases faibles NaHCO₃ ou KHCO₃ par 2 équivalents de bases faibles plus basiques comme Na₂CO₃ ou K₂CO₃ conduit à une augmentation significative des vitesses de réactions qui sont complètes en un peu plus de 2 h 30 avec un rendement quantitatif en FDCA. L'ajout de 4 équivalents de Na₂CO₃ accélère encore la réaction qui est alors complète en moins de 2 heures.

### Exemple 6 (selon l'invention) : comparaison entre 2 modes de préparations des catalyseurs bimétalliques PtBi/C

L'activité de deux catalyseurs bimétalliques préparés selon deux modes, ex-situ et in-situ, a été comparée dans les conditions standards de la réaction avec un rapport Na₂C0₃/HMF de 2. La totalité du bismuth ajouté a été déposée sur le catalyseur monométallique Pt/C dans le cas où un sel de bismuth soluble en milieu alcalin (comme le (Bi(NO₃)₃, 5H₂O) est utilisé. Les tableaux 21 et 22 résument les résultats obtenus avec les deux différents modes de préparations :

**Tableau 21**

| **3,6%Pt0,68%Bi/C(L3S) - in-situ Bi/Pt=0,19** | | | | | | |
|---|---|---|---|---|---|---|
| **Durée [h]** | **HMF [mM]** | **%C HMF** | **FFCA [mM]** | **%R FFCA** | **FDCA [mM]** | **%R FDCA** |
| 0 | 98 | 0 | 0 | 0 | 0 | 0 |
| 0,25 | 13 | 87 | 38 | 39 | 31 | 32 |
| 0,5 | 0 | 100 | 24 | 25 | 67 | 68 |
| 1 | 0 | 100 | 11 | 12 | 83 | 85 |
| 2 | 0 | 100 | 4 | 4 | 93 | 94 |
| 3 | 0 | 100 | 1 | 1 | 96 | 98 |
| 4 | 0 | 100 | 0 | 0 | 98 | 100 |
| 5 | 0 | 100 | 0 | 0 | 98 | 100 |
| 6 | 0 | 100 | 0 | 0 | 98 | 100 |

**Tableau 22**

| **5,1%Pt1%Bi/C(3SW) - ex-situ Bi/Pt=0,2** | | | | | | |
|---|---|---|---|---|---|---|
| **Durée [h]** | **HMF [mM]** | **%C HMF** | **FFCA [mM]** | **%R FFCA** | **FDCA [mM]** | **%R FDCA** |
| 0 | 96 | 0 | 0 | 0 | 0 | 0 |
| 0,16 | 8 | 92 | 59 | 62 | 17 | 18 |
| 0,33 | 0 | 100 | 42 | 44 | 47 | 49 |
| 0,5 | 0 | 100 | 29 | 30 | 63 | 66 |
| 1 | 0 | 100 | 16 | 17 | 79 | 83 |
| 2 | 0 | 100 | 4 | 4 | 91 | 95 |
| 3 | 0 | 100 | 0 | 0 | 95 | 99 |
| 4 | 0 | 100 | 0 | 0 | 95 | 100 |
| 6 | 0 | 100 | 0 | 0 | 96 | 100 |

Les résultats montrent que quels que soient les modes de préparation du catalyseur bimétallique, à condition de choisir un sel précurseur de Bi soluble en milieu alcalin, l'activité du catalyseur bimétallique reste comparable.

### Exemple 7 (selon l'invention) : Influence de la température

Le procédé tel que décrit ci-dessus a été mis en oeuvre avec le catalyseur 5,1%Pt-1%Bi/C(3SW) (Bi/Pt = 0,2), avec un ratio molaire Na₂CO₃/HMF = 2 et une température comprise entre 80 et 120°C.

Les résultats sont regroupés dans les tableaux 23 à 25 ci-dessous.

**Tableau 23**

| **100°C** | | | | | | |
|---|---|---|---|---|---|---|
| **Durée [h]** | **HMF [mM]** | **%C HMF** | **FFCA [mM]** | **%R FFCA** | **FDCA [mM]** | **%R FDCA** |
| 0 | 96 | 0 | 41 | 43 | 0 | 0 |
| 0,1667 | 8 | 91 | 59 | 62 | 17 | 18 |
| 0,3333 | 0 | 100 | 42 | 44 | 47 | 49 |
| 0,5 | 0 | 100 | 29 | 30 | 63 | 66 |
| 1 | 0 | 100 | 16 | 17 | 79 | 83 |
| 2 | 0 | 100 | 4 | 4 | 91 | 95 |
| 3 | 0 | 100 | 0 | 0 | 95 | 99 |
| 4 | 0 | 100 | 0 | 0 | 95 | 100 |
| 5 | 0 | 100 | 0 | 0 | 96 | 100 |
| 6 | 0 | 100 | 0 | 0 | 96 | 100 |
| 10 | 0 | 100 | 0 | 0 | 96 | 100 |

**Tableau 24**

| **80°C** | | | | | | |
|---|---|---|---|---|---|---|
| **Durée [h]** | **HMF [mM]** | **%C HMF** | **FFCA [mM]** | **%R FFCA** | **FDCA [mM]** | **%R FDCA** |
| 0 | 100 | 0 | 0 | 0 | 0 | 0 |
| 0,25 | 20 | 80 | 50 | 50 | 18 | 18 |
| 0,5 | 0 | 100 | 46 | 46 | 43 | 43 |
| 1 | 0 | 100 | 29 | 29 | 65 | 65 |
| 2 | 0 | 100 | 14 | 14 | 83 | 83 |
| 3 | 0 | 100 | 9 | 9 | 90 | 90 |
| 4 | 0 | 100 | 4 | 4 | 95 | 95 |
| 6 | 0 | 100 | 1 | 1 | 98 | 98 |
| 8 | 0 | 100 | 0 | 0 | 99 | 99 |
| 10 | 0 | 100 | 0 | 0 | 99 | 99 |
| 12 | 0 | 100 | 0 | 0 | 99 | 99 |

**Tableau 25**

| **120°C** | | | | | | |
|---|---|---|---|---|---|---|
| **Durée [h]** | **HMF [mM]** | **%C HMF** | **FFCA [mM]** | **%R FFCA** | **FDCA [mM]** | **%R FDCA** |
| 0 | 97 | 0 | 0 | 0 | 0 | 0 |
| 0,1 | 38 | 61 | 43 | 44 | 4 | 4 |
| 0,2 | 5 | 95 | 50 | 51 | 37 | 38 |
| 0,4 | 0 | 100 | 22 | 23 | 73 | 76 |
| 0,6 | 0 | 100 | 10 | 11 | 87 | 90 |
| 0,8 | 0 | 100 | 6 | 6 | 91 | 93 |
| 1 | 0 | 100 | 3 | 3 | 94 | 97 |
| 1,5 | 0 | 100 | 1 | 1 | 95 | 98 |
| 2 | 0 | 100 | 0 | 0 | 96 | 99 |
| 2,5 | 0 | 100 | 0 | 0 | 97 | 100 |
| 3 | 0 | 100 | 0 | 0 | 97 | 100 |
| 5 | 0 | 100 | 0 | 0 | 96 | 99 |
| 12 | 0 | 100 | 0 | 0 | 96 | 99 |

Une augmentation de la température permet d'augmenter la vitesse de réaction à condition de ne pas dépasser une température de 160°C, au-delà de laquelle les réactions de dégradation de l'HMF peuvent avoir lieu.

### Exemple 8 (selon l'invention) : Influence de la pression

Le procédé tel que décrit ci-dessus a été mis en oeuvre avec le catalyseur 5,1%Pt-1%Bi/C(3SW) (Bi/Pt = 0,2), avec un ratio molaire Na₂CO₃/HMF = 2 à une température de 100°C à une pression de 50 bar et 60 bar d'air.

**Tableau 26**

| **50bar** | | | | | | |
|---|---|---|---|---|---|---|
| **Durée [h]** | **HMF [mM]** | **%C HMF** | **FFCA [mM]** | **%R FFCA** | **FDCA [mM]** | **%R FDCA** |
| 0 | 99 | 0 | 0 | 0 | 0 | 0 |
| 0,25 | 26 | 76 | 35 | 35 | 27 | 28 |
| 0,5 | 0 | 100 | 31 | 32 | 63 | 64 |
| 1 | 0 | 100 | 14 | 15 | 83 | 84 |
| 2 | 0 | 100 | 6 | 6 | 92 | 93 |
| 3 | 0 | 100 | 2 | 2 | 97 | 98 |
| 4 | 0 | 100 | 0 | 0 | 98 | 99 |
| 5 | 0 | 100 | 0 | 0 | 98 | 99 |
| 6 | 0 | 100 | 0 | 0 | 98 | 99 |
| 10 | 0 | 100 | 0 | 0 | 98 | 99 |

Les résultats obtenus à 60 bar d'air sont similaires à ceux obtenus à 50 bar et montrent qu'une pression au-delà de 40 bar n'a pas d'influence sur la réaction.

### Exemple 9 (selon l'invention) : Recyclage du catalyseur bimétallique PtBi

La stabilité du catalyseur 3.6%Pt-0,9%Bi/C(L3S), (Bi/Pt = 0,25) a été étudiée en présence de NaHCO₃ comme base avec un ratio molaire NaHCO₃/HMF de 4) en recyclant le catalyseur trois fois: 1^{er} cycle avec le catalyseur neuf puis 3 cycles distincts avec le même catalyseur après lavage à l'eau et séchage à l'étuve à 100°C à l'air sans traitement de réduction. Les résultats sont regroupés dans les tableaux ci-dessous

**Tableau 27**

| **1^{er} cycle** | | | | | | |
|---|---|---|---|---|---|---|
| **Durée [h]** | **HMF [mM]** | **% HMF** | **FFCA [mM]** | **% FFCA** | **FDCA [mM]** | **% FDCA** |
| 0 | 103 | 0 | 0 | 0 | 0 | 0 |
| 0,16 | 55 | 47 | 29 | 28 | 1 | 1 |
| 0,33 | 13 | 87 | 64 | 63 | 8 | 8 |
| 0,5 | 0 | 100 | 70 | 68 | 18 | 18 |
| 0,75 | 0 | 100 | 63 | 61 | 29 | 29 |
| 1 | 0 | 100 | 56 | 55 | 38 | 37 |
| 2 | 0 | 100 | 38 | 36 | 60 | 59 |
| 3 | 0 | 100 | 19 | 18 | 80 | 78 |
| 4 | 0 | 100 | 10 | 10 | 89 | 86 |
| 6 | 0 | 100 | 3 | 3 | 96 | 94 |
| 8 | 0 | 100 | 1 | 1 | 99 | 96 |
| 10 | 0 | 100 | 0 | 0 | 100 | 97 |
| 12 | 0 | 100 | 0 | 0 | 101 | 98 |

**Tableau 28**

| **2^{ème} cycle** | | | | | | |
|---|---|---|---|---|---|---|
| **Durée [h]** | **HMF [mM]** | **% HMF** | **FFCA [mM]** | **% FFCA** | **FDCA [mM]** | **% FDCA** |
| 0 | 100 | 10 | 0 | 0 | 0 | 0 |
| 0,25 | 64 | 36 | 19 | 19 | 1 | 1 |
| 0,5 | 22 | 78 | 53 | 53 | 7 | 7 |
| 0,75 | 4 | 96 | 66 | 66 | 16 | 16 |
| 1 | 0 | 100 | 64 | 63 | 24 | 24 |
| 2 | 0 | 100 | 47 | 47 | 47 | 47 |
| 3 | 0 | 100 | 33 | 33 | 65 | 64 |
| 4,1 | 0 | 100 | 22 | 22 | 76 | 76 |
| 6 | 0 | 100 | 12 | 12 | 88 | 87 |
| 8 | 0 | 100 | 4 | 4 | 97 | 96 |
| 10,1 | 0 | 100 | 1 | 1 | 99 | 99 |
| 12 | 0 | 100 | 0 | 0 | 100 | 100 |

**Tableau 29**

| **3^{ème} cycle** | | | | | | |
|---|---|---|---|---|---|---|
| **Durée [h]** | **HMF [mM]** | **% HMF** | **FFCA [mM]** | **% FFCA** | **FDCA [mM]** | **% FDCA** |
| 0 | 100 | 0 | 0 | 0 | 0 | 0 |
| 0,25 | 68 | 32 | 17 | 17 | 1 | 1 |
| 0,5 | 28 | 72 | 49 | 49 | 6 | 6 |
| 0,75 | 4 | 96 | 66 | 66 | 17 | 17 |
| 1 | 0 | 100 | 63 | 63 | 25 | 25 |
| 2 | 0 | 100 | 48 | 47 | 48 | 48 |
| 3 | 0 | 100 | 33 | 33 | 66 | 66 |
| 4 | 0 | 100 | 23 | 23 | 76 | 76 |
| 6 | 0 | 100 | 12 | 12 | 90 | 90 |
| 8,1 | 0 | 100 | 4 | 4 | 97 | 97 |
| 10 | 0 | 100 | 2 | 2 | 100 | 100 |
| 12 | 0 | 100 | 0 | 0 | 100 | 100 |

**Tableau 30**

| **4^{ème} cycle** | | | | | | |
|---|---|---|---|---|---|---|
| **Durée [h]** | **HMF [mM]** | **% HMF** | **FFCA [mM]** | **% FFCA** | **FDCA [mM]** | **% FDCA** |
| 0 | 99 | 0 | 0 | 0 | 0 | 0 |
| 0,25 | 72 | 27 | 15 | 15 | 1 | 1 |
| 0,5 | 44 | 56 | 38 | 38 | 4 | 4 |
| 0,75 | 16 | 84 | 61 | 62 | 11 | 11 |
| 1 | 2 | 98 | 68 | 69 | 19 | 19 |
| 2 | 0 | 100 | 60 | 60 | 35 | 36 |
| 3 | 0 | 100 | 48 | 48 | 48 | 49 |
| 4 | 0 | 100 | 38 | 38 | 59 | 60 |
| 6 | 0 | 100 | 21 | 22 | 76 | 76 |
| 8 | 0 | 100 | 11 | 11 | 87 | 88 |
| 10 | 0 | 100 | 5 | 5 | 93 | 94 |
| 12 | 0 | 100 | 0 | 0 | 98 | 99 |

Les résultats montrent que le catalyseur bimétallique PtBi/C ne perd pas significativement son activité, contrairement au catalyseur monométallique Pt/C (exemple 3) qui nécessite une nouvelle réduction avant chaque réutilisation pour qu'il garde une bonne activité catalytique.

### Exemple 10 (selon l'invention) : Influence de la masse du catalyseur

Le procédé tel que décrit ci-dessus a été mis en oeuvre à 120°C en utilisant deux fois moins de catalyseur (HMF/Pt = 200) en présence de 2 équivalents de Na₂CO₃ avec le catalyseur 5,1%Pt1%Bi/C(3SW). Le rendement en FDCA est toujours supérieur à 95% avec un temps de réaction de moins de 8 h.

## Revendications

1. Procédé de préparation d'acide 2,5-furane dicarboxylique (FDCA) par oxydation du 5-hydroxyméthylfurfural (HMF) dans l'eau en présence d'une base faible et d'un catalyseur supporté comprenant du platine et du bismuth, dans lequel le ratio molaire Bi/Pt dans le catalyseur est compris entre 0,1 à 0,3.

2. Procédé selon la revendication 1, dans lequel le milieu réactionnel ne comprend pas de solvant organique.

3. Procédé selon l'une des revendications 1 ou 2, dans lequel la quantité de platine dans le catalyseur est comprise entre 1 % et 10 % en poids par rapport au poids de catalyseur supporté.

4. Procédé selon l'une quelconque des revendications 1 à 3, dans lequel la base faible est choisie parmi les bases dont le pKb est supérieur à 1,4.

5. Procédé selon l'une quelconque des revendications 1 à 4, dans lequel la base faible est choisie parmi les carbonates et hydrogénocarbonates de métaux alcalins, seuls ou en mélange.

6. Procédé selon l'une quelconque des revendications 1 à 5, dans lequel le procédé est mis en oeuvre sous pression de façon à avoir un ratio molaire O₂/HMF supérieur à 2.

7. Procédé selon l'une quelconque des revendications 1 à 6 mené à une température de 70 à 150°C.

8. Procédé selon l'une quelconque des revendications 1 à 7, dans lequel le ratio molaire base faible/HMF est supérieur ou égal à 1.

9. Procédé selon l'une quelconque des revendications 1 à 8, dans lequel le ratio molaire HMF/Pt est compris entre 50 et 400.

10. Procédé selon l'une quelconque des revendications 1 à 9, dans lequel le ratio molaire Bi/Pt dans le catalyseur est compris entre 0,15 et 0,3.

11. Procédé selon l'une quelconque des revendications 1 à 10, dans lequel la quantité de platine dans le catalyseur est comprise entre 3% et 6% en poids par rapport au poids de catalyseur supporté.

12. Procédé selon l'une quelconque des revendications 1 à 11, dans lequel la base faible est choisie parmi les carbonates et hydrogénocarbonates de sodium et potassium.

13. Procédé selon l'une quelconque des revendications 1 à 12, mené à une température de 80 à 120°C.

## Patentansprüche

1. Verfahren zur Herstellung von 2,5-Furandicarbonsäure (FDCA) durch Oxidation von 5-Hydroxymethylfurfural (HMF) in Wasser in der Gegenwart einer schwachen Base und einem Trägerkatalysator umfassend Platin und Bismut, wobei das molare Verhältnis Bi/Pt in dem Katalysator zwischen 0,1 und 0,3 beträgt.

2. Verfahren gemäß Anspruch 1, worin das Reaktionsmedium kein organisches Lösungsmittel umfasst.

3. Verfahren gemäß einem der Ansprüche 1 oder 2, worin die Menge von Platin in dem Katalysator zwischen 1 Gewichts-% und 10 Gewichts-% beträgt, bezogen auf das Gewicht des Trägerkatalysators.

4. Verfahren gemäß einem der Ansprüche 1 bis 3, worin die schwache Base aus den Basen, deren pKb höher als 1,4 ist, ausgewählt ist.

5. Verfahren gemäß einem der Ansprüche 1 bis 4, worin die schwache Base ausgewählt ist aus Carbonaten und Hydrogencarbonaten von Akalimetallen, entweder allein oder als Mischung.

6. Verfahren gemäß einem der Ansprüche 1 bis 5, worin das Verfahren durchgeführt wird unter Druck, um ein molares Verhältnis O₂/HMF von höher als 2 zu erhalten.

7. Verfahren gemäß einem der Ansprüche 1 bis 6, durchgeführt bei einer Temperatur von 70 bis 150 °C.

8. Verfahren gemäß einem der Ansprüche 1 bis 7, wobei das molare Verhältnis schwache Base/HMF höher oder gleich 1 ist.

9. Verfahren gemäß einem der Ansprüche 1 bis 8, wobei das molare Verhältnis HMF/Pt zwischen 50 und 400 beträgt.

10. Verfahren gemäß einem der Ansprüche 1 bis 9, wobei das molare Verhältnis Bi/Pt in dem Katalysator zwischen 0,15 und 0,3 beträgt.

11. Verfahren gemäß einem der Ansprüche 1 bis 10, wobei die Menge des Platins im Katalysator zwischen 3 Gewichts-% und 6 Gewichts-% beträgt, bezogen auf das Gewicht des Trägerkatalysators.

12. Verfahren gemäß einem der Ansprüche 1 bis 11, wobei die schwache Base ausgewählt ist aus Carbonaten und Hydrogencarbonaten von Natrium und Kalium.

13. Verfahren gemäß einem der Ansprüche 1 bis 12, durchgeführt bei einer Temperatur von 80 bis 120 °C.

## Claims

1. A method for preparing 2,5-furandicarboxylic acid (FDCA) by oxidising 5-hydroxymethylfurfural (HMF) in water in the presence of a weak base and a supported catalyst comprising platinum and bismuth, wherein the Bi/Pt molar ratio in the catalyst is between 0.1 and 0.3.

2. The method according to claim 1 wherein the reaction medium does not comprise an organic solvent.

3. The method according to one of claims 1 or 2 wherein the amount of platinum in the catalyst is between 1 % and 10 % by weight relative to the weight of the supported catalyst.

4. The method according to any of claims 1 to 3 wherein the weak base is selected from among bases having a pKb value higher than 1.4.

5. The method according to any of claims 1 to 4 wherein the weak base is selected from among the carbonates and hydrogen carbonates of alkaline metals, either alone or in a mixture.

6. The method according to any of claims 1 to 5 wherein the method is implemented under pressure so as to obtain a O₂/HMF molar ratio higher than 2.

7. The method according to any of claims 1 to 6 conducted at a temperature of 70 to 150°C.

8. The method according to any of claims 1 to 7 wherein the weak base/HMF molar ratio is 1 or higher.

9. The method according to any of claims 1 to 8 wherein the HMF/Pt molar ratio is between 50 and 400.

10. The method according to any of claims 1 to 9 wherein the Bi/Pt molar ratio in the catalyst is between 0.15 and 0.3.

11. The method according to any of claims 1 to 10 wherein the amount of platinum in the catalyst is between 3 % and 6 % by weight relative to the weight of the supported catalyst.

12. The method according to any of claims 1 to 11 wherein the weak base is selected from among the carbonates and hydrogen carbonates of sodium and potassium.

13. The method according to any of claims 1 to 12 conducted at a temperature of 80 to 120°C.
